# DEMANDE DE BREVET EUROPEEN

(11) **EP 4 837 898 A1**
(43) Date de publication de la demande: **11.11.2026**
(21) Numéro de dépôt: 26176703.2
(22) Date de dépôt: 05.05.2026
(51) Int. Cl.: C07C 323/67, C07C 319/22, C07C 319/24, C07C 323/65

(54) **COMPOSES DISULFURES ET LEUR PROCEDE D OBTENTION**

(30) Priorité: 06.05.2025 FR 2504813
(71) Demandeur: Commissariat à l'Energie Atomique et aux Energies Alternatives, 75015 Paris (FR); Université Grenoble Alpes, 38401 Saint-Martin-d'Hères (FR); Centre National de la Recherche Scientifique, 75016 Paris (FR)
(72) Inventeur: FARINA, Chloé, 38054 Grenoble cedex 09 (FR); BERNARD, Laurent, 38054 Grenoble cedex 09 (FR); LANDA, Matthieu, 38054 Grenoble cedex 09 (FR); PICARD, Lionel, 38054 Grenoble cedex 09 (FR); LECONTE, Nicolas, 38401 SAINT-MARTIN D'HERES (FR)
(74) Mandataire: Gevers & Orès

(57) **Abrégé**

La présente invention concerne des nouveaux composés disulfures et leur procédé d'obtention, ces composés étant des précurseurs de thiols, utilisés notamment dans des réactions radicalaires (thiolène), des additions et des substitutions nucléophiles.

## Description

La présente invention concerne des nouveaux composés disulfures et leur procédé d'obtention, ces composés étant des précurseurs de thiols, utilisés notamment dans des réactions radicalaires (thiol-ène), des additions et des substitutions nucléophiles.

Les électrolytes polymères sont composés d'une matrice polymère dans laquelle a été dissout un sel de lithium. Dans ce système, tous les ions, peu importe leur nature, sont conduits : les ions Li⁺ et leurs contre-ions migrent entre l'électrode positive et l'électrode négative de la cellule au cours des processus charge-décharge. Ce phénomène génère peu à peu des gradients de concentration des ions entrainant une accumulation de charges en surface des électrodes, responsable de la polarisation de la cellule et associée à la croissance dendritique.

L'une des stratégies pour s'affranchir de la polarisation de la cellule électrochimique, consiste à greffer de façon covalente le contre-ion du Li⁺ sur les chaines macromoléculaires, de manière à ce que seul le Li⁺ se déplace au sein du polymère. La diffusion de l'anion est donc stoppée. Ces électrolytes polymères ainsi caractérisés par leur nombre de transport proche de l'unité sont dits « à conduction unipolaire » ou « single-ion », en anglais. La majorité des électrolytes polymères à conduction unipolaire étudiés dans la littérature comportent des groupements anioniques de type carboxylates, sulfonates ou imides dérivés des sels classiquement utilisés dans les électrolytes liquides (TFSI, FSI, etc.).

Dans la littérature, le thiol d'intérêt est obtenu en 4 étapes à partir de l'allylsulfonate de sodium, comme indiqué ci-après. Ce thiol est mis en jeu dans la synthèse d'électrolyte polymères à conduction unipolaire à base de polyéthers (POE). Cette synthèse permet d'obtenir le thiol d'intérêt avec de 15 à 43 % de rendement.

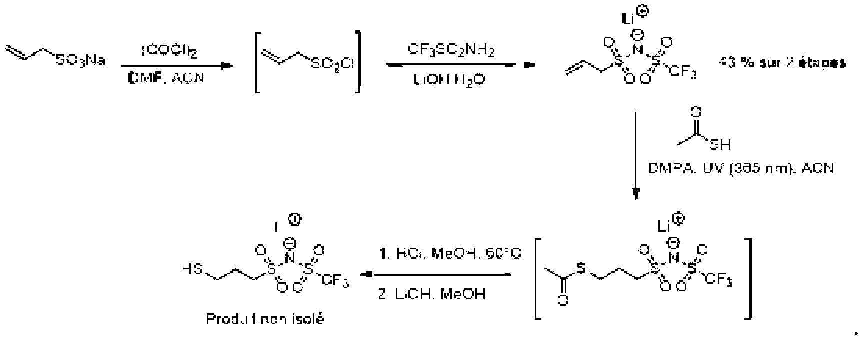

Aussi, un objectif de l'invention est de mettre à disposition un composé cible pouvant être utilisé en tant que précurseur de thiol pour des réactions radicalaires (thiol-ène), d'additions et de substitutions nucléophiles. Dans le domaine des batteries Li-ion, ce composé cible permet de greffer le groupement trifluorométhanesulfonylimidure de lithium sur des monomères ou des polymères et générer des électrolytes polymères à conduction unipolaire (« single-ion »). En outre, ce composé cible peut être, selon l'invention, isolé, et également obtenu avec une grande pureté.

Un autre objectif de l'invention est celui de mettre à disposition ce composé d'intérêt à l'aide d'un procédé d'obtention simple, économique et robuste. En particulier, le composé d'intérêt est obtenu selon le procédé de l'invention avec un rendement d'au moins 62 % sur l'ensemble de la synthèse, à partir du 3-mercapto-1-propanesulfonate de sodium ou du dimère correspondant, accessibles commercialement et/ou peu coûteux (et plus accessibles commercialement et/ou moins coûteux que l'allylsulfonate de sodium décrit dans l'art antérieur).

Aussi, l'invention concerne un composé de formule (I) suivante : 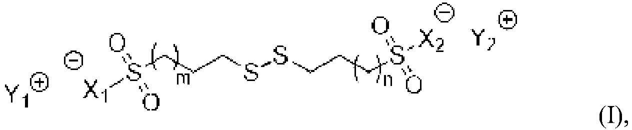 dans laquelle :
m et n représentent indépendamment un nombre entier de 0 à 10, en particulier 1 ;
X₁ et X₂ sont indépendamment choisis parmi les groupes suivants : 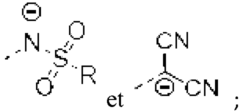
avec R étant indépendamment choisi parmi :
   - les groupes alkyles linéaires ou ramifiés en C₁ à C₆ fluorés ou perfluorés, en particulier perfluorés, par exemple CF₃ ou CF₂-CF₃ ;
   - F ;
   - les groupes alkyles linéaires ou ramifiés en C₁ à C₆, en particulier méthyle ;
Y₁ et Y₂ sont indépendamment choisis parmi Li, Na et K.

Le procédé de l'invention permet une protection des thiols qui est conservée dans le composé cible de l'invention, car la fonction disulfure correspondante peut être réduite *in situ* en thiolate dans bon nombre des réactions subséquentes visées.

Ainsi, m et n représentent indépendamment 0, 1, 2, 3, 4, 5, 6, 7, 8, 9 ou 10.

Selon un mode de réalisation particulier, m et n représentent indépendamment un nombre entier de 0 à 5, en particulier de 0 à 3, plus particulièrement de 0 à 2, par exemple 1.

Selon un mode de réalisation particulier, m et n représentent indépendamment un nombre entier de 1 à 5, en particulier de 1 à 3, plus particulièrement de 1 à 2, par exemple 1.

Selon un mode de réalisation particulier, le composé de formule (I) est de formule (Ia) suivante : 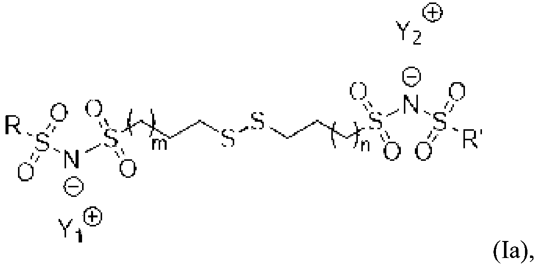
dans laquelle R et R' sont indépendamment choisis parmi :
   - les groupes alkyles linéaires ou ramifiés en C₁ à C₆ fluorés ou perfluorés, en particulier perfluorés, par exemple CF₃ ou CF₂-CF₃ ;
   - F ;
   - les groupes alkyles linéaires ou ramifiés en C₁ à C₆, en particulier méthyle ;
en particulier de formule (Ia') suivante :

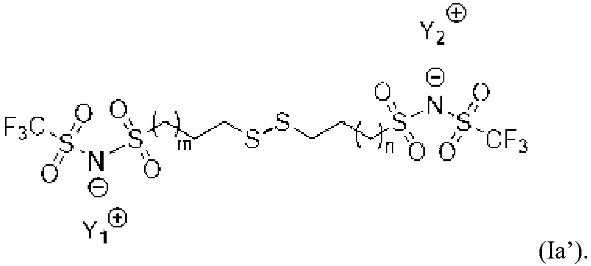

Selon un mode de réalisation particulier, le composé de formule (I) est de formule (Ib) suivante :

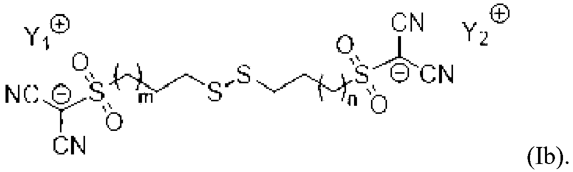

Selon un mode de réalisation particulier, m et n sont identiques.

Selon un mode de réalisation particulier, X₁ et X₂ sont identiques.

Selon un mode de réalisation particulier, Y₁ et Y₂ sont identiques.

Selon un mode de réalisation particulier :
- m et n sont identiques ;
- X₁ et X₂ sont identiques ; et
- Y₁ et Y₂ sont identiques.

Selon un mode de réalisation particulier, m et n ne représentent pas 0.

Selon un mode de réalisation particulier, m et n sont de 1.

Selon un mode de réalisation particulier, Y₁ et Y₂ représentent Li.

Selon un mode de réalisation particulier, Y₁ et Y₂ représentent Na.

Selon un mode de réalisation particulier, Y₁ et Y₂ représentent K.

Selon un mode de réalisation particulier, m et n ne représentent pas 0, m et n étant notamment de 1, Y₁ et Y₂ représentant Li.

Selon un mode de réalisation particulier, m et n ne représentent pas 0, m et n étant notamment de 1, Y₁ et Y₂ représentant Na.

Selon un mode de réalisation particulier, m et n ne représentent pas 0, m et n étant notamment de 1, Y₁ et Y₂ représentant K.

Selon un mode de réalisation particulier, le composé de formule (I) est de l'une des formules suivantes :

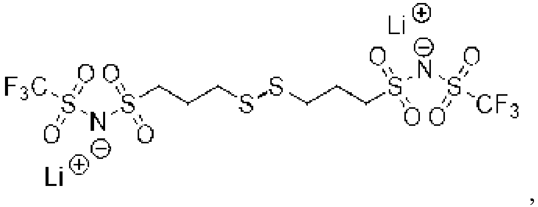

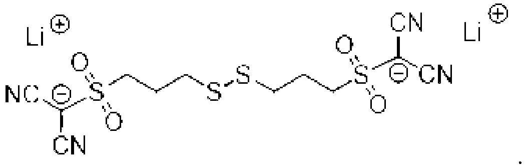

Selon un autre aspect, la présente invention concerne également un procédé de préparation d'un composé de formule (I) tel que défini précédemment, comprenant les étapes suivantes :
(i) Une étape d'halogénation d'un composé de formule (II) suivante pour obtenir un composé de formule (III) suivante : 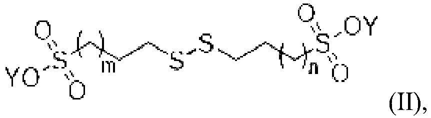 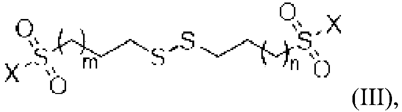 Où Y représente un métal alcalin, en particulier Na, K ou Li; et X est un halogène, en particulier Cl ou Br ;
(ii) Une étape de mise en contact du composé (III) tel qu'obtenu à l'issue de l'étape précédente avec un composé de formule (IVa) suivante : R-SO₂-NH₂ , et/ou un composé de formule (IVb) suivante, R'-SO₂-NH₂, et/ou avec du malononitrile, en présence d'une base B, B étant une amine, pour obtenir un composé de formule (V) suivante : 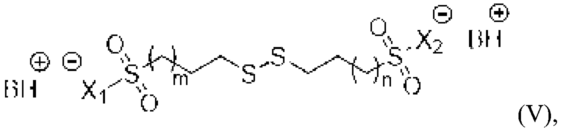
(iii) Une étape d'échange des ions BH⁺ du composé de formule (V) tel qu'obtenu à l'issue de l'étape précédente par des ions Y₁⁺ et/ou Y₂⁺ pour obtenir ledit composé de formule (I).

Selon un mode de réalisation particulier, l'étape (ii) est alternativement comme suit: (ii) Une étape de mise en contact du composé (III), tel qu'obtenu à l'issue de l'étape précédente, avec NH₃, puis avec un composé de formule (IVa') R-SO₂-Cl et/ou un composé de formule (IVb') R'-SO₂-Cl, en présence d'une base B, B étant une amine,
pour obtenir un composé de formule (V) suivante :

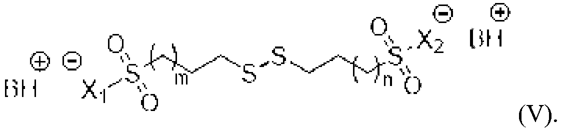

Certains des composés de formule (II) sont commerciaux.

Les composés peuvent également être obtenus selon le mode de réalisation suivant.

Selon un mode de réalisation particulier, l'étape (i) est précédée d'une étape (o) :
- d'oxydation d'un composé de formule (VI) suivante : 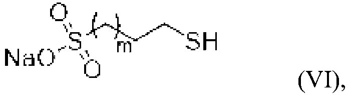 Ou
- De formation d'un disulfure à partir d'un composé de formule (VIa) et d'un composé de formule (VIb) de formules suivantes : 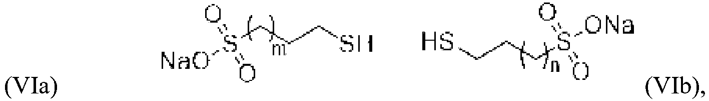 Pour obtenir ledit composé de formule (II).

Selon un mode de réalisation particulier, le procédé tel que défini précédemment comprend les étapes suivantes :
(i) Une étape d'halogénation d'un composé de formule (II) suivante pour obtenir un composé de formule (III) suivante : 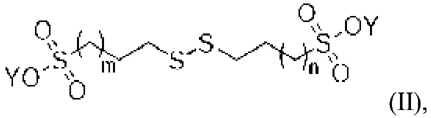 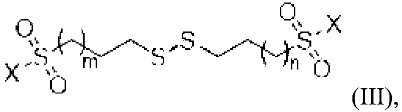 Où Y représente un métal alcalin, en particulier Na, K ou Li; et X est un halogène, en particulier Cl ou Br, m et n étant identiques ;
(ii) Une étape de mise en contact du composé (III) tel qu'obtenu à l'issue de l'étape précédente avec un composé de formule (IVa) suivante : R-SO₂-NH₂ ou avec du malononitrile, en présence d'une base B, B étant une amine, pour obtenir un composé de formule (V) suivante : 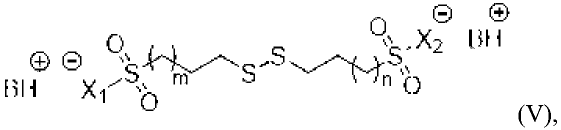 X₁ et X₂ étant identiques, m et n étant identiques ;
(iii) Une étape d'échange des ions BH⁺ du composé de formule (V) tel qu'obtenu à l'issue de l'étape précédente par des ions Y₁⁺ pour obtenir ledit composé de formule (I), dans lequel Y₁⁺ et Y₂⁺ sont identiques, X₁ et X₂ étant identiques, m et n étant identiques.

Selon un mode de réalisation particulier, l'étape (ii) est alternativement comme suit: :
(ii) Une étape de mise en contact du composé (III), tel qu'obtenu à l'issue de l'étape précédente, avec NH₃, puis avec un composé de formule (IVa') R-SO₂-Cl, en présence d'une base B, B étant une amine,

Pour obtenir un composé de formule (V) suivante : 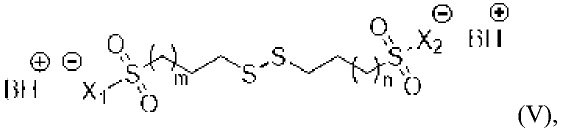 X₁ et X₂ étant identiques, m et n étant identiques.

Selon un mode de réalisation particulier, l'étape (i) est précédée d'une étape (o) de dimérisation d'un composé de formule (VI) suivante : 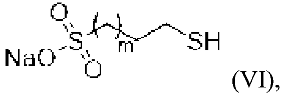 pour obtenir ledit composé de formule (II).

L'étape (o) peut être réalisée par toute technique de dimérisation bien connue de l'homme du métier, notamment en présence de diiode, de dioxygène, de peroxyde d'hydrogène, ou de diacétate d'iodobenzène.

Selon un mode de réalisation particulier, le composé de formule (II) est de formule particulière suivante :

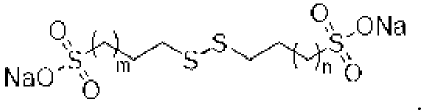

Selon un mode de réalisation particulier, l'étape (i) d'halogénation est une étape de chloration, et X est Cl.

Selon un mode de réalisation plus particulier, l'étape (i) est effectuée par mise en contact du composé de formule (IIa) avec du chlorure de thionyle, du chlorure d'oxalyle ou un halogénure de phosphore, par exemple PCl₅ ou PCl₃, en particulier du chlorure de thionyle.

Selon un autre mode de réalisation particulier, l'étape (i) d'halogénation est une étape de bromation, et X est Br.

Selon un mode de réalisation plus particulier, l'étape (i) est effectuée par mise en contact du composé de formule (IIa) avec du bromure de thionyle, du bromure d'oxalyle ou un bromure de phosphore, par exemple PBr₅ ou PBr₃, en particulier du bromure de thionyle.

Selon un mode de réalisation particulier, la base B est choisie parmi triéthylamine, 1,8-diazabicyclo[5.4.0]undéc-7-ène (DBU), 1,5,7-triazabicyclo[4.4.0]déc-5-ène (TBD).

Selon un mode de réalisation particulier, l'étape (ii) est réalisée en présence d'un catalyseur, par exemple le 4-diméthylaminopyridine (DMAP).

L'étape (iii) d'échange d'ions peut être réalisée par toute technique bien connue de l'homme du métier, par exemple :
- à l'aide d'une résine échangeuse d'ions, qu'elle soit commerciale ou non, en particulier une résine échangeuse de cations fortement acides (SAC), plus particulièrement une résine polymérique avec des groupes fonctionnels acide sulfonique, par exemple conditionnée par LiOH, et en particulier éluée à l'aide d'eau, d'un mélange acétone : eau, ou d'un mélange méthanol : eau ; ou
- par mise en contact avec une solution de K₂CO₃, Li₂CO₃ ou Na₂CO₃ dans un solvant organique, par exemple l'acétonitrile, comme par exemple décrit par Ho et al. (Polym. Chem. 8 (2017) 5660-5665).

De façon particulièrement avantageuse, le suivi de l'étape (iii) et son achèvement peuvent être effectués par RMN, en particulier du proton, en observant la disparation des signaux correspondant aux ions BH⁺ du composé (V).

### DEFINITIONS

Tel qu'on l'entend ici, les plages de valeur sous forme de « x-y » ou « de x à y » ou « entre x et y » incluent les bornes x et y, les entiers compris entre ces bornes, ainsi que tous les autres nombres réels compris entre ces bornes. A titre d'exemple, « 1-5 », ou « de 1 à 5 » ou « entre 1 et 5 » désignent les entiers 1, 2, 3, 4 et 5, ainsi que tous les autres nombres réels compris entre 1 et 5. Les modes de réalisations préférés incluent chaque entier pris individuellement dans la plage de valeur, ainsi que toute sous-combinaison de ces entiers et tout ensemble de nombres réels compris entre ces entiers. A titre d'exemple, les valeurs préférées pour « 1-5 » peuvent comprendre les entiers 1, 2, 3, 4, 5, 1-2, 1-3, 1-4, 1-5, 2-3, 2-4, 2-5, etc.

Tel qu'on l'utilise dans la présente description, le terme « environ » se réfère à un intervalle de valeurs de ± 10 % d'une valeur spécifique. A titre d'exemple, l'expression « environ 20 » comprend les valeurs de 20 ± 10 %, soit les valeurs de 18 à 22.

Tel qu'il est utilisé ici, le terme "alkyle" désigne un groupe alkyle à chaîne linéaire ou ramifiée ayant le nombre d'atomes de carbone indiqué avant ledit terme, notamment 1 à 8 atomes de carbone, tel que méthyle, éthyle, propyle, isopropyle, butyle, isobutyle, sec-butyle, tert-butyle, pentyle, isoamyle, néopentyle, 1-éthylpropyle, 3-méthylpentyle, 2,2-diméthylbutyle, 2,3-diméthylbutyle, hexyle, octyle, etc. Ainsi, une expression telle que "alkyle en C1-C4" désigne un radical alkyle contenant de 1 à 4 atomes de carbone. Il en est de même pour le terme « alcane ».

### EXEMPLES

### Exemple 1 : Synthèse de composés de l'invention

Les réactifs et solvants commerciaux ont été utilisés sans purification supplémentaire. Le trifluorométhanesulfonamide a été acheté auprès de ABCR. Les autres réactifs et solvants ont été achetés chez Sigma-Aldrich. La résine échangeuse d'ions Amberlite IR-120 sous forme acide, achetée auprès de Sigma-Aldrich, a été convertie en sa forme lithiée par agitation dans une solution aqueuse de LiOH (10 eq.) pendant une nuit. La résine a ensuite été lavée à l'eau distillée jusqu'à pH égal à 7, pour retirer l'excès de LiOH. Les spectres RMN ¹H, ¹⁹F, et ¹³C ont été réalisés sur un spectromètre Bruker NEO 400MHz équipé d'une sonde 5 mm BBO Smartprobe. Les spectres de RMN ¹H et ¹³C ont été référencés au pic du solvant. Les spectres de RMN ¹⁹F ont été référencés au pic de l'hexafluorobenzène (C₆F₆) utilisé comme standard interne. Les spectres de masses ont été enregistrés avec un spectromètre Thermo Scientific LTQ Orbitrap XL. Les analyses élémentaires CHNS ont été effectuées à l'aide d'un analyseur élémentaire organique Elementar Unicube.

La méthode de synthèse exemplifiée est présentée ci-après :

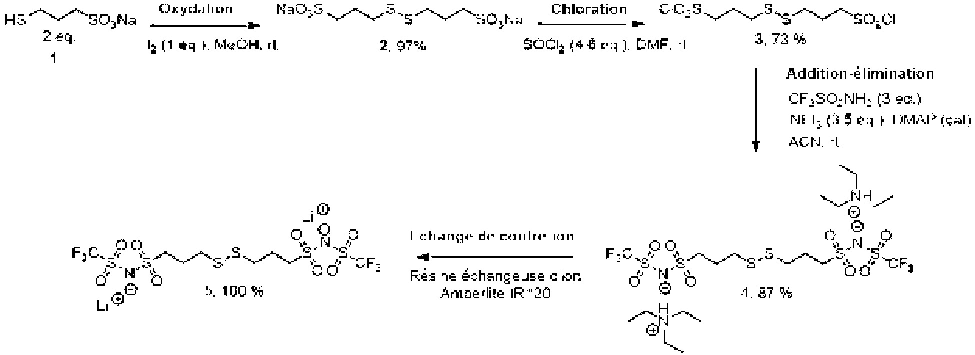

Dans une première étape, le disulfure **2** a été préparé par oxydation du thiol **1** en présence de diiode en milieu basique. Cette étape permet de protéger la fonction thiol afin qu'elle ne réagisse pas lors des étapes suivantes de la synthèse. Le produit **2** a été isolé avec 97 % de rendement. Dans une seconde étape, une chloration des groupes sulfonates de **2** a été réalisée par réaction avec du chlorure de thionyle dans le DMF anhydre à température ambiante. Le produit **3** a été isolé avec 73 % de rendement. Dans une troisième étape, le composé **4** a été préparé à température ambiante par réaction entre **3** et le trifluorométhanesulfonamide en présence de DMAP et de triéthylamine dans l'acétonitrile anhydre. Le disulfure sous forme de sel de triéthylamine **4** a été isolé avec 87 % de rendement. Enfin le composé **4** a été converti en son sel de lithium après passage sur une résine échangeuse d'ions lithiée.

Cette méthode a permis d'obtenir le composé **5** pur avec 62 % de rendement sur l'ensemble de la synthèse. Un des avantages de cette méthode est que la lithiation peut être suivie par disparition de l'ion triéthylammonium, visible en RMN du proton. Il n'y a donc pas d'ambiguïtés sur la nature du contre-ion. Cette méthode peut aussi être facilement mise en place pour générer le sel de Na et K.

### Synthèse de 2 :

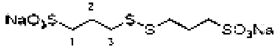

A température ambiante, du diiode (10.71 g, 42.2 mmol, 1.0 eq.) a été ajouté sous agitation à une solution de 3-mercapto-1-propanesulfonate (15.03 g, 84.4 mmol, 2.0 eq.) dans le méthanol (500 mL). Une fois le diiode complètement dissous, de la soude (3.38 g, 84.4 mmol, 2.0 eq.) a été ajoutée au milieu réactionnel. Celui-ci a été laissé à température ambiante pendant 18 h. Le milieu réactionnel a ensuite été filtré sur fritté et le solide a été lavé à l'acétone. Le solide blanc récolté correspondant à **2** a été séché sous vide (14.45 g, Rdt = 97 %).

### Synthèse de 3 :

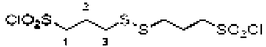

Sous atmosphère d'argon, du chlorure de thionyle (9.5 mL, 131.2 mmol, 4.6 eq.) a été ajouté au goutte à goutte à une solution de **2** (10.11 g, 28.5 mmol, 1 eq.) dans le DMF anhydre (40 mL) préalablement refroidie à 0 °C. Une fois l'addition terminée, le milieu réactionnel a été laissé revenir à température ambiante et agité pendant 18 h. Le milieu réactionnel a ensuite été versé dans de l'eau distillée froide (400 mL). La phase aqueuse a été extraite avec de l'acétate d'éthyle (150 mL). La phase organique a ensuite été lavée à deux reprises avec une solution aqueuse de LiCl (5%) ainsi qu'une fois avec une solution aqueuse saturée en NaCl. La phase organique a été séchée sur MgSO₄, filtrée et séchée sous vide pour donner une huile brune correspondant à **3** (7.23 g, Rdt = 73 %).

RMN ¹H (400 MHz, CDCl₃) : δ (ppm) : 3.85-3.82 (m, 4H, H₁), 2.87 (t, *³J* = 6.8 Hz, 4H, H₃), 2.52-2.45 (m, 4H, H₂). RMN ¹³C (100 MHz, CDCl₃) : δ (ppm) : 63.4 (C₁), 35.2 (C₃), 23.7 (C₂). Anal. élem. calculée pour C₆H₁₂O₄S₄Cl₂ : C 20.75, H 3.48, S 36.93 % ; Trouvée : C 21.02, H 3.47, S 36.82 %.

### Synthèse de 4 :

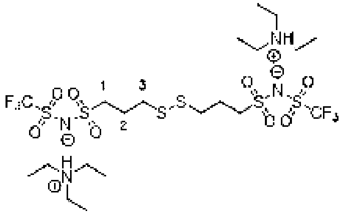

Dans un ballon bicol sous atmosphère d'argon, ont été introduits successivement du trifluorométhanesulfonamide (11,83 g, 79.3 mmol, 3 eq.), de l'acétonitrile anhydre (12 mL), de la DMAP (0.10 g, 0.8 mmol, 0.3 eq.) ainsi que de la triéthylamine (13 mL, 93.3 mmol, 3.5 eq.). Une solution de 3 (9.16 g, 26.4 mmol, 1 eq.) dans l'acétonitrile anhydre (8 mL) a ensuite été ajoutée sous agitation au milieu réactionnel, au goutte à goutte à 0 °C. Une fois l'addition terminée, le milieu réactionnel a été laissé revenir à température ambiante et agité pendant 18 h. Le milieu réactionnel a ensuite été filtré sur fritté, concentré sous vide. Le brut réactionnel a été purifié par colonne de gel de silice (Eluant : CH₂Cl₂/MeOH ; 100 : 0 jusqu'à 85 : 15 (v : v)) pour donner une huile brune correspondant à **4** (17.34 g, Rdt = 87 %). R_{f} = 0.24 (CH₂Cl₂/MeOH ; 9 : 1 (v : v)). RMN ¹H (400 MHz, CDCl₃) : δ (ppm) : 3.32-3.28 (m, *³J* = 7.4 Hz, 4H, H₁), 3.23-3.16 (m, 12H, TEA), 2.83 (t, *³J* = 7.1 Hz, 4H, H₃), 2.29-2.22 (m, 4H, H₂), 1.36 (t, *³J* = 7.3 Hz, 18H, TEA). RMN ¹³C (100 MHz, CDCl₃) : δ (ppm) : 120.3 (q, *¹J_{C-F} =* 323 Hz, C₄), 53.5 (C₁), 46.7 (TEA), 37.1 (C₃), 23.7 (C₂), 8.7 (TEA). RMN ¹⁹F (376 MHz, CDCl₃) : δ (ppm) : 83.9 (CF₃). HRMS (ESI) : m/z calc. pour C₁₄H₂₈F₆N₃O₈S₆ : 672.01103 [M-(C₆H₁₆N)]⁻; trouvé : 672.00923.

### Synthèse de 5 :

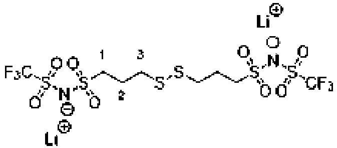

**4** (17.22 g, 22.3 mmol, 1 eq.) a été dissous dans un mélange acétone : eau (1 : 1) (v : v). La solution a ensuite été passée une fois au travers d'une première colonne de résine échangeuse d'ions Amberlite^{™} IR120-H préalablement lithiée et neutralisée (130 mL, 10 eq.) puis trois fois dans une deuxième colonne de même nature (130 mL). La colonne a ensuite été lavée plusieurs fois avec de l'éluant pour récupérer la totalité du produit. Après évaporation de l'acétone sous vide et lyophilisation, le sel de lithium **5** a été obtenu (13.17 g, Rdt = 100 %).

RMN ¹H (400 MHz, DMSO-d₆) : δ (ppm) : 3.07-3.03 (m, 4H, H₁), 2.81 (t, *³J* = 7.2 Hz, 4H, H₃), 2.06-1.99 (m, 4H, H₂). RMN ¹³C (100 MHz, DMSO-d₆) : δ (ppm) : 120.2 (q, *¹J_{C-F} =* 324 Hz, C₄), 53.0 (C₁), 35.8 (C₃), 23.5 (C₂). RMN ¹⁹F (376 MHz, DMSO-d₆) : δ (ppm) : 85.0 (-CF₃). HRMS (ESI) : m/z calc. pour C₈H₁₂F₆N₂O₈S₆Li : 576.89877 [M-Li]⁻; trouvé : 576.89685.

### Exemple 2 : Synthèse avec le malononitrile

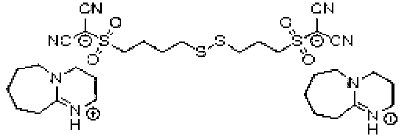

Dans un ballon bicol sous atmosphère d'argon, sont introduits successivement du malononitrile (0.57 g, 8.64 mmol, 3 eq.), de l'acétonitrile anhydre (3 mL) ainsi que du DBU (1.53 g, 10.0 mmol, 3.5 eq.). Une solution de **3** (1 g, 2.88 mmol, 1 eq.) dans l'acétonitrile anhydre (3 mL) est ensuite ajoutée sous agitation au milieu réactionnel, au goutte à goutte à 0 °C. Une fois l'addition terminée, le milieu réactionnel est laissé revenir à température ambiante et agité pendant 18 h. Le milieu réactionnel est ensuite filtré sur fritté, concentré sous vide. Le brut réactionnel est purifié par colonne de gel de silice.

### Exemple 3 : Echange d'ions avec K₂CO₃

A une solution refroidie de **4** (2 g, 2.68 mmol, 1 eq.) dans de l'acétonitrile (12 ml), est ajouté lentement du carbonate de potassium (0.93 g, 6.7 mmol, 2.5 eq.). Après l'addition complète de K₂CO₃, le mélange est laissé réagir entre 0°C et la température ambiante pendant 24 heures. La solution est ensuite filtrée et le filtrat est concentré sous vide. Le solide est recristallisé à partir de dichlorométhane chaud. La solution du mélange est ensuite conservée à -20°C pendant 24 heures. Le solide obtenu est lavé avec du n-pentane froid et séché sous vide.

Cet exemple peut également être réalisé avec Li₂CO₃ ou Na₂CO₃.

## Revendications

1. Composé de formule (I) suivante : 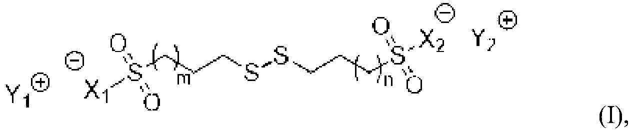 dans laquelle :
m et n représentent indépendamment un nombre entier de 0 à 10, en particulier 1 ;
X₁ et X₂ sont indépendamment choisis parmi les groupes suivants : 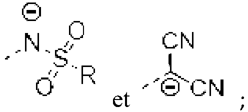
avec R étant indépendamment choisi parmi :
- les groupes alkyles linéaires ou ramifiés en C₁ à C₆ fluorés ou perfluorés, en particulier perfluorés, par exemple CF₃ ou CF₂-CF₃ ;
- F ;
- les groupes alkyles linéaires ou ramifiés en C₁ à C₆, en particulier méthyle ;
Y₁ et Y₂ sont indépendamment choisis parmi Li, Na et K.

2. Composé selon la revendication 1, de formule (Ia) suivante : 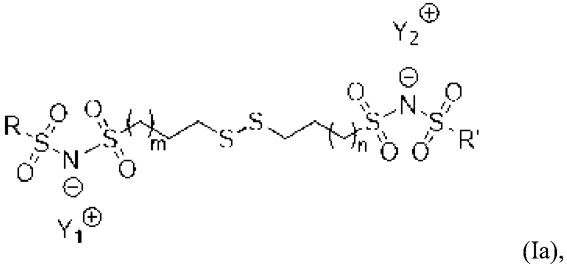
dans laquelle R et R' sont indépendamment choisis parmi :
- les groupes alkyles linéaires ou ramifiés en C₁ à C₆ fluorés ou perfluorés, en particulier perfluorés, par exemple CF₃ ou CF₂-CF₃ ;
- F ;
- les groupes alkyles linéaires ou ramifiés en C₁ à C₆, en particulier méthyle ;
en particulier de formule (Ia') suivante :

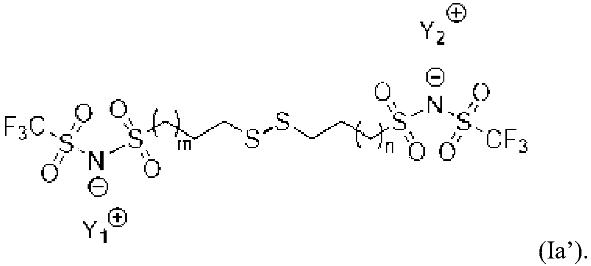

3. Composé selon la revendication 1, de formule (Ib) suivante :

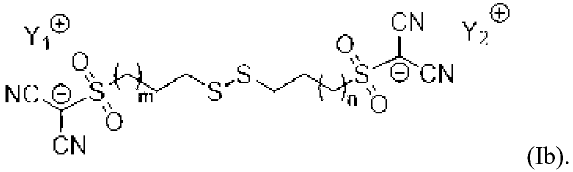

4. Composé selon l'une quelconque des revendications précédentes, de l'une des formules suivantes :

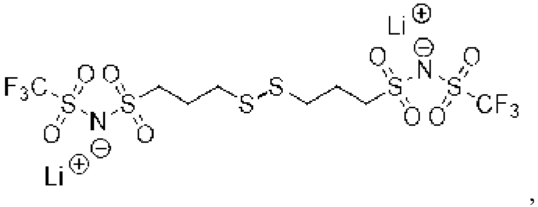

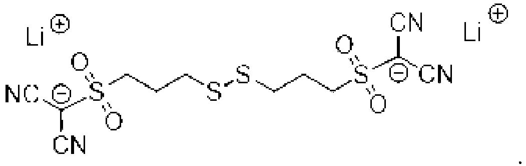

5. Procédé de préparation d'un composé de formule (I) selon l'une quelconque des revendications 1 à 4, comprenant les étapes suivantes :
(i) Une étape d'halogénation d'un composé de formule (II) suivante pour obtenir un composé de formule (III) suivante : 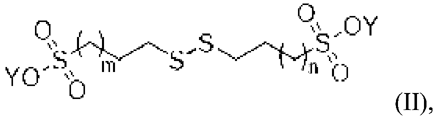 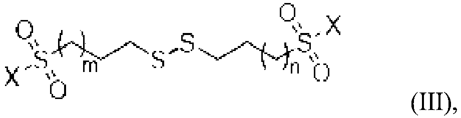 Où Y représente un métal alcalin, en particulier Na, K ou Li; et X est un halogène, en particulier Cl ou Br ;
(ii) Une étape de mise en contact du composé (III) tel qu'obtenu à l'issue de l'étape précédente avec un composé de formule (IVa) suivante : R-SO₂-NH₂, et/ou un composé de formule (IVb) suivante, R'-SO₂-NH₂, et/ou avec du malononitrile, en présence d'une base B, B étant une amine, pour obtenir un composé de formule (V) suivante : 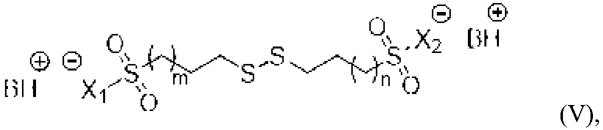
(iii) Une étape d'échange des ions BH⁺ du composé de formule (V) tel qu'obtenu à l'issue de l'étape précédente par des ions Y₁⁺ et/ou Y₂⁺ pour obtenir ledit composé de formule (I).

6. Procédé selon la revendication 5, dans lequel l'étape (ii) est alternativement comme suit:
(ii) Une étape de mise en contact du composé (III), tel qu'obtenu à l'issue de l'étape précédente, avec NH₃, puis avec un composé de formule (IVa') R-SO₂-Cl et/ou un composé de formule (IVb') R'-SO₂-Cl, en présence d'une base B, B étant une amine,
pour obtenir un composé de formule (V) suivante :

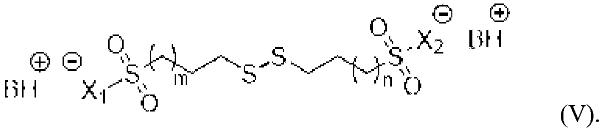

7. Procédé selon l'une quelconque des revendications 5 à 6, dans lequel l'étape (i) est précédée d'une étape (o) :
- d'oxydation d'un composé de formule (VI) suivante : 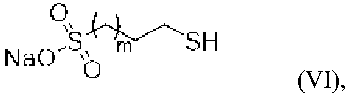 Ou
- De formation d'un disulfure à partir d'un composé de formule (VIa) et d'un composé de formule (VIb) de formules suivantes : 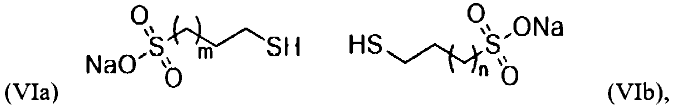 Pour obtenir ledit composé de formule (II).

8. Procédé selon l'une quelconque des revendications 5 à 7 de préparation d'un composé de formule (I) selon l'une quelconque des revendications 1 à 4 comprenant les étapes suivantes :
(i) Une étape d'halogénation d'un composé de formule (II) suivante pour obtenir un composé de formule (III) suivante : 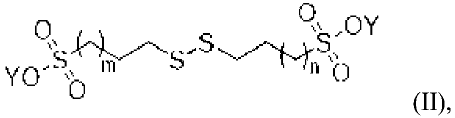 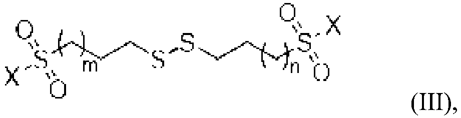 Où Y représente un métal alcalin, en particulier Na, K ou Li; et X est un halogène, en particulier Cl ou Br, m et n étant identiques ;
(ii) Une étape de mise en contact du composé (III) tel qu'obtenu à l'issue de l'étape précédente avec un composé de formule (IVa) suivante : R-SO₂-NH₂ ou avec du malononitrile, en présence d'une base B, B étant une amine, pour obtenir un composé de formule (V) suivante : 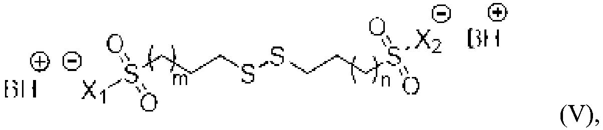 X₁ et X₂ étant identiques, m et n étant identiques ;
(iii) Une étape d'échange des ions BH⁺ du composé de formule (V) tel qu'obtenu à l'issue de l'étape précédente par des ions Y₁⁺ pour obtenir ledit composé de formule (I), dans lequel Y₁⁺ et Y₂⁺ sont identiques, X₁ et X₂ étant identiques, m et n étant identiques.

9. Procédé selon la revendication 8, dans lequel l'étape (ii) est alternativement comme suit::
(ii) Une étape de mise en contact du composé (III), tel qu'obtenu à l'issue de l'étape précédente, avec NH₃, puis avec un composé de formule (IVa') R-SO₂-Cl, en présence d'une base B, B étant une amine,
Pour obtenir un composé de formule (V) suivante : 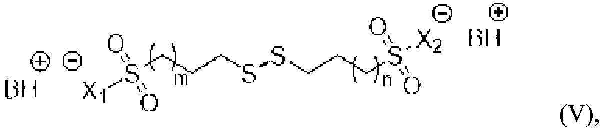 X₁ et X₂ étant identiques, m et n étant identiques.

10. Procédé selon la revendication 8 ou 9, dans lequel l'étape (i) est précédée d'une étape (o) de dimérisation d'un composé de formule (VI) suivante : 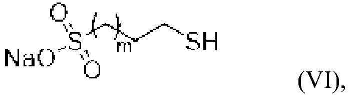 pour obtenir ledit composé de formule (II).
